# EUROPÄISCHE PATENTANMELDUNG

(11) **EP 3 492 049 A1**
(43) Veröffentlichungstag der Anmeldung: **05.06.2019**
(21) Anmeldenummer: 17200855.9
(22) Anmeldetag: 09.11.2017
(51) Int. Cl.: A61F 5/01

(54) **STÜTZSCHALENANORDNUNG**

(71) Anmelder: Natsis, Michael, 8142 Uitikon-Waldegg (CH); Gomez Diaz, Juan, 9496 Balzers (LI)
(72) Erfinder: Natsis, Michael, 8142 Uitikon-Waldegg (CH); Gomez Diaz, Juan, 9496 Balzers (LI)
(74) Vertreter: Rutz, Andrea

(57) **Zusammenfassung**

Eine Stützschalenanordnung (1) umfasst ein Wadenteil (2), ein Fussteil (3), und eine Feststelleinrichtung (5) mit einer Führungsschiene (52) sowie mit einem Feststellelement (51). Das Feststellelement (51) ist zum Einstellen eines Beugewinkels (a) zwischen dem Wadenteil (2) und dem Fussteil (3) entlang der Führungsschiene (52) verschiebbar und in einer gewünschten Position fixierbar. Die Feststelleinrichtung (5) umfasst zudem ein Federelement (53), welches das Feststellelement (51) derart mit einer Federkraft beaufschlagt, dass ein Verschieben des Feststellelements (51) entlang der Führungsschiene (52) erleichtert oder erschwert ist. Eine Einlage (7) für eine Stützschalenanordnung umfasst eine erste Kammer (71) und eine zweite Kammer (72). Mittels einer ersten Ventileinrichtung (73) ist ein Fluid in die erste Kammer einfüllbar, um die Einlage (7) in einem ersten Bereich zu versteifen. Mittels einer zweiten Ventileinrichtung (74) ist ein Fluid aus der mit Formkörpern (77) gefüllten zweiten Kammer (72) abführbar, um die Einlage (7) in einem zweiten Bereich zu versteifen.

## Beschreibung

### TECHNISCHES GEBIET

Die vorliegende Erfindung betrifft eine Stützschalenanordnung sowie eine Einlage, insbesondere einen Innenschuh, für eine Stützschalenanordnung.

### STAND DER TECHNIK

Zur Behandlung von Extremitätenfrakturen wird heutzutage oftmals auf einen Gipsverband verzichtet. So liegt ein Nachteil eines Gipsverbandes darin, dass ein an einer Extremität ausgehärteter Gips nachträglich nicht mehr an seiner Passform geändert werden kann. Bei einem zu engen Gips besteht dann die Gefahr, dass Druckstellen entstehen und keine ausreichende Durchblutung der Extremität mehr erfolgen kann.

Insbesondere bei der Behandlung von Verletzungen von Gelenken des menschlichen Körpers oder von Gliedmassen, die durch Gelenke miteinander verbunden sind, ist es üblich, das Gelenk und die entsprechende Gliedmasse während der Genesungs- und Rehabilitationszeit mittels einer abnehmbaren Stütze ruhigzustellen. Der Stand der Technik weist eine grosse Anzahl von unterschiedlichen derartigen orthopädischen Vorrichtungen auf. So sind Stützen bekannt, welche die Gliedmassen und das Gelenk in einer fixierten Position relativ zueinander halten, oder solche, die ein Gelenk aufweisen, welches eine Beugung oder Bewegungsfreiheit zulässt. Stützen, welche aus mehreren Schalenteilen bestehen, ermöglichen eine flexible Anpassung an die zu behandelnde Extremität. Wesentlich ist dabei allerdings eine hohe Genauigkeit bei der Anpassung der Schalenteile an die Extremität, da der Heilungsprozess vor allem dann gefördert wird, wenn die Stütze eine anatomisch korrekte Stellung der zu behandelnden Extremität bewirkt.

Beispielsweise ist aus der WO 2009/112164 A1 eine Stützschalenanordnung mit einem Wadenteil zur Aufnahme eines Unterschenkels und einem über eine Gelenkanordnung mit dem Wadenteil verbundenen Fussteil bekannt. Weiter ist fersenseitig eine Beugewinkel-Einstelleinrichtung vorgesehen, mittels welcher ein gewünschter Beugewinkel zwischen dem Fussteil und dem Wadenteil eingestellt werden kann, wobei eine Fixierung über eine Drehverriegelung erfolgt.

Das Anlegen und insbesondere das genaue Anpassen einer Stütze erfordern allerdings ein erhebliches Geschick und einen entsprechenden Aufwand für das medizinische Personal bzw. den Patienten.

Damit die Stützen beziehungsweise Stützschalenanordnungen die notwendige Stabilität erreichen, weisen diese üblicherweise eine gewisse Eigensteifigkeit auf. Jeder Patient besitzt jedoch eine sehr individuelle Körperform, so dass trotz flexiblen Stützvorrichtungen oftmals Druckstellen entstehen, was für den Patienten unbequem und auch schmerzhaft sein kann.

### DARSTELLUNG DER ERFINDUNG

In einem ersten Aspekt ist es daher eine Aufgabe der vorliegenden Erfindung, eine Stützschalenanordnung anzugeben, welche eine flexible und genaue Anpassung an eine zu behandelnde Extremität ermöglicht und gleichzeitig über eine einfache Handhabung verfügt.

Diese Aufgabe wird mit einer Stützschalenanordnung gemäss Anspruch 1 gelöst.

In einem zweiten Aspekt ist es eine Aufgabe der vorliegenden Erfindung, eine Einlage für eine Stützschalenanordnung anzugeben, welche über einen hohen Tragekomfort verfügt.

Diese Aufgabe wird mit einer Einlage gemäss Anspruch 15 gelöst.

Gemäss einem ersten Aspekt wird also eine Stützschalenanordnung angegeben, welche ein Wadenteil, ein Fussteil, welches gelenkig mit dem Wadenteil verbindbar ist, und eine Feststelleinrichtung mit einer am Wadenteil oder am Fussteil angebrachten Führungsschiene sowie mit einem Feststellelement umfasst. Das Feststellelement ist zum Einstellen eines Beugewinkels zwischen dem Wadenteil und dem Fussteil entlang der Führungsschiene verschiebbar und in einer gewünschten Position an der Führungsschiene fixierbar. Die Feststelleinrichtung umfasst zudem ein Federelement, welches das Feststellelement derart mit einer Federkraft beaufschlagt, dass ein Verschieben des Feststellelements entlang der Führungsschiene erleichtert oder erschwert ist.

Aufgrund der gelenkigen Verbindung zwischen dem Wadenteil und dem Fussteil ist eine beliebig einstellbare Beugung beziehungsweise Neigung des Wadenteils bezüglich des Fussteils möglich.

Oder anders gesagt, das Wadenteil kann gegenüber dem Fussteil verschwenkt werden. Unter dem Beugewinkel wird in diesem Zusammenhang der Winkel verstanden, welcher zwischen einer Hauptlängsachse des Wadenteils und einer Hauptlängsachse des Fussteils gebildet wird und das Ausmass der Beugung oder Verschwenkung zwischen dem Wadenteil und dem Fussteil charakterisiert. Dabei können unterschiedliche Beugewinkel in einem Bereich von zum Beispiel 75° bis 120° eingestellt werden. Mögliche Beugewinkel betragen dann beispielsweise 120°, 115°, 100°, 95°, 90°, 85°, 80°, 75°, wobei 90° einem Wadenteil entspricht, welches im Wesentlichen senkrecht zum Fussteil steht. Ein Beugewinkel von 100° bedeutet, dass das Wadenteil ausgehend von seiner senkrechten Position um 10° in Richtung der Ferse gebeugt wurde, und ein Beugewinkel von 60° bedeutet, dass das Wadenteil ausgehend von seiner senkrechten Position um 30° in Richtung der Zehen gebeugt wurde. Diese Beugungsfähigkeit ermöglicht, dass die Stützschalenanordnung eine anatomisch korrekte Stellung einnehmen kann.

Damit der Beugewinkel eingestellt werden kann, weist die Stützschalenanordnung eine Feststelleinrichtung auf, deren Führungsschiene entweder am Wadenteil oder am Fussteil angebracht ist, und deren Feststellelement entlang der Führungsschiene verschiebbar und in einer gewünschten Position an der Führungsschiene fixierbar ist. Unter der gewünschten Position wird hier insbesondere diejenige Position bezeichnet, welche der anatomisch korrekten Stellung entspricht, also einem bestimmten Beugewinkel zwischen dem Wadenteil und dem Fussteil.

Die Führungsschiene kann über eine Einraststruktur und das Feststellelement kann über eines oder mehrere Einrastelemente verfügen, wobei das Feststellelement bewegbar ist zwischen einer Verschiebeposition, in welcher das oder die Einrastelemente ausser Eingriff mit der Einraststruktur stehen, und einer Fixierposition, in welcher das oder die Einrastelemente in Eingriff mit der Einraststruktur stehen.

Die Federkraft des Federelements kann in einer ersten Ausführungsform bewirken, dass ein Verschieben des Feststellelements entlang der Führungsschiene erleichtert ist, solange das Feststellelement zum Beispiel mittels eines Fixierelements nicht an der Führungsschiene fixiert ist. Auf diese Weise kann das Einstellen des gewünschten Beugewinkels zwischen Waden- und Fussteil für den Patienten bzw. für das medizinischen Personal erleichtert werden, da der Kraftaufwand zum Bewegen des Wadenteils relativ zum Fussteil dann aufgrund des Federelements verringert ist. Ausserdem kann die durch das Federelement bewirkte erleichterte Bewegung zwischen dem Waden- und dem Fussteil dem Patienten in einer fortgeschritteneren Therapiephase auch eine gewisse Bewegungsfreiheit ermöglichen. Aufgrund der gelenkigen Verbindung zwischen dem Waden- und dem Fussteil wird dem Patienten dann eine geführte Bewegung zwischen Unterschenkel und Fuss erlaubt.

Um ein Verschieben des Feststellelements entlang der Führungsschiene zu erleichtern, wird das Feststellelement vom Federelement bei dieser ersten Ausführungsform bevorzugt mit einer Federkraft in Richtung der Verschiebeposition beaufschlagt. Die Federkraft bewirkt dabei vorteilhaft, dass das oder die Einrastelemente des Feststellelements ausser Eingriff mit der Einraststruktur der Führungsschiene stehen.

Es ist also insbesondere möglich, eine Rastverbindung zwischen der Führungsschiene und dem Feststellelement vorzusehen, wobei eine Kraftbeaufschlagung des Feststellelements durch das Federelement dazu führt, dass das oder die Einrastelemente des Feststellelements ausser Eingriff mit der Einraststruktur der Führungsschiene gebracht werden. Das Feststellelement befindet sich dann aufgrund der Federkraft in der Verschiebeposition und wird durch das Federelement in dieser gehalten. Wirkt eine der Federkraft entgegengerichtete Kraft auf das Feststellelement, deren Betrag grösser als der Betrag der Federkraft ist, so wird das Feststellelement entgegen der Federkraft von der Verschiebeposition in die Fixierposition überführt, wobei das oder die Einrastelemente des Feststellelements in Eingriff mit der Einraststruktur der Führungsschiene gelangen. Eine derartige entgegengerichtete Kraft kann insbesondere durch ein Fixierelement bewirkt werden.

In einer alternativen zweiten Ausführungsform kann die Federkraft des Federelements aber auch bewirken, dass nach Einstellen des gewünschten Beugewinkels zwischen Waden- und Fussteil ein Verschieben des Feststellelements entlang der Führungsschiene erschwert ist, dass also das Feststellelement und dadurch auch die Stützschalenanordnung weitgehend in der gewünschten Position verbleiben bevor das Feststellelement an der Führungsschiene fixiert wird. Vor der Fixierung des Feststellelements an der Führungsschiene kann dadurch das Risiko eines ungewollten Verschiebens der Stützschalenanordnung aus der anatomisch korrekten Stellung heraus reduziert oder sogar vermieden werden, wodurch eine genaue Anpassung der Stützschalenanordnung an die zu behandelnde Extremität gewährleistet wird. Das Feststellelement wird mittels des Federelements also gewissermassen in der gewünschten Position an der Führungsschiene vorfixiert. Da diese Vorfixierung durch die Federkraft des Feststellelements erfolgt, geschieht die Vorfixierung in gewissem Sinne automatisch. Automatisch heisst hier, dass das Federelement ohne weiteres Zutun von einem Benutzer oder unter Verwendung von Hilfsmitteln oder zusätzlichen Verriegelungsstrukturen bewirkt, dass ein unbeabsichtigtes Verschieben des Feststellelements und dadurch ein unbeabsichtigtes Verändern des Beugewinkels der Stützschalenanordnung weitgehend vermieden wird. Dadurch wird eine einfache Handhabung bei der Einstellung des Beugewinkels der Stützschalenanordnung gewährleistet.

Um bei dieser alternativen zweiten Ausführungsform ein Verschieben des Feststellelements entlang der Führungsschiene zu erschweren, wird das Feststellelement bevorzugt vom Federelement mit einer Federkraft in Richtung der Fixierposition beaufschlagt. Die Federkraft bewirkt dabei vorteilhaft die oben erwähnte automatische Vorfixierung.

Es ist also denkbar, eine Rastverbindung zwischen der Führungsschiene und dem Feststellelement vorzusehen, wobei eine Kraftbeaufschlagung des Feststellelements durch das Federelement dazu führt, dass das oder die Einrastelemente des Feststellelements mit der Einraststruktur der Führungsschiene in Eingriff gebracht werden. Dann ist das Feststellelement an der Führungsschiene vorfixiert und das Feststellelement befindet sich in der Fixierposition. Wirkt eine der Federkraft entgegengerichtete Kraft auf das Feststellelement, deren Betrag grösser als der Betrag der Federkraft ist, so wird das Feststellelement entgegen der Federkraft von der Fixierposition in die Verschiebeposition überführt, wobei der Eingriff zwischen dem oder den Einrastelementen des Feststellelements und der Einraststruktur der Führungsschiene gelöst wird. Will also beispielsweise ein Arzt eine anatomisch korrekte Stellung der Stützschalenanordnung einstellen, so drückt er das Feststellelemente entgegen der Federkraft ausser Eingriff mit der Führungsschiene und verschwenkt das Wadenteil relativ zum Fussteil respektive das Fussteil relativ zum Wadenteil, bis diese im gewünschten Beugewinkel zueinander stehen. Befinden sich das Wadenteil und das Fussteil in einem gewünschten Beugewinkel zueinander, so lässt der Arzt das Feststellelement los, wodurch die Federkraft das Feststellelement automatisch in Eingriff mit der Führungsschiene bringt und die Stützschalenanordnung im gewünschten Beugewinkel vorfixiert.

Es gilt allerdings zu verstehen, dass eine Rastverbindung bei beiden erwähnten Ausführungsformen nicht zwingend vorhanden sein muss. Stattdessen ist es denkbar, dass das Feststellelement einzig derart ausgebildet ist, dass es die Führungsschiene hintergreifen kann, ohne jedoch eine Rastverbindung einzugehen. In der Fixierposition wird dann zum Beispiel ein Anschlag zwischen den Bereichen des Feststellelements, welche die Führungsschiene hintergreifen, und den Bereichen der Führungsschiene, welche vom Feststellelement hintergriffen werden, gebildet. Zwischen den sich dabei berührenden Bereichen des Feststellelements und der Führungsschiene bildet sich aufgrund der Wirkung des Federelements eine Haftreibung aus, welche ein Verschieben des Feststellelements entlang der Führungsschiene zumindest erschwert oder sogar verunmöglicht. Wird bei der ersten Ausführungsform, bei welcher die Federkraft das Verschieben des Feststellelements erleichtert, eine der Federkraft entgegenwirkende Kraft auf das Feststellelement ausgeübt, so wird das Feststellelement in Anschlag mit der Führungsschiene gebracht, so dass eine Haftreibung zwischen dem Feststellelement und der Führungsschiene entsteht und ein Verschieben des Feststellelements in dieser entsprechend eingenommenen Fixierposition erschwert ist. Wird bei der zweiten Ausführungsform, bei welcher die Federkraft das Verschieben des Feststellelements erschwert, eine der Federkraft entgegenwirkende Kraft auf das Feststellelement ausgeübt, so wird die Haftreibung zwischen der Führungsschiene und dem Feststellelement verringert oder ganz aufgehoben. Das Feststellelement befindet sich dann in der Verschiebeposition und ist entlang der Führungsschiene verschiebbar.

Die Feststelleinrichtung kann weiter über zumindest ein Fixierelement, insbesondere in Form einer Fixierschraube, zur Fixierung des Feststellelements in der gewünschten Position an der Führungsschiene verfügen.

Das heisst, eine zusätzliche Fixierung des Feststellelements in der Fixierposition kann durch das Vorsehen eines Fixierelements bewirkt werden, welches je nach Ausführungsform eine der Federkraft entgegengerichtete oder gleichgerichtete Fixierkraft auf das Feststellelement ausüben kann. Vorteilhaft bewirkt das Fixierelement auf das Feststellelement bei der ersten Ausführungsform eine der Federkraft entgegengerichtete Fixierkraft und bei der zweiten Ausführungsform eine der Federkraft gleichgerichtete Fixierkraft.

Das Fixierelement kann bei der ersten Ausführung zum Beispiel in Form einer Fixierschraube vorgesehen sein, welche in ein am Feststellelement ausgebildetes Innengewinde einschraubbar ist, um das Feststellelement entgegen die vom Federelement bewirkte Federkraft in Richtung der Fixierposition zu bringen.

Bei der zweiten Ausfiihrungsform kann das Feststellelement beispielsweise eine Durchgangsöffnung für eine Fixierschraube aufweisen, durch welche die Fixierschraube eingeführt und in Richtung der Führungsschiene geschraubt wird, bis dass sie auf der Führungsschiene auftrifft. Dabei wird von der Fixierschraube eine Fixierkraft auf das Feststellelement erzeugt, welche das Feststellelement analog zum Federelement mit einer Kraft beaufschlägt und dieses so in die Fixierposition überführt respektive in der Fixierposition fixiert. Diese zusätzlich zur Federkraft wirkende Fixierkraft verkleinert das Risiko einer ungewollten Verschiebung des Wadenteils entlang der Führungsschiene in der Fixierposition, wodurch die Sicherheit der Stützschalenanordnung bzgl. des eingestellten Beugewinkels weiter erhöht wird.

Das Fussteil kann fersenseitig gebogen ausgebildet sein und mit diesem gebogenen Bereich zumindest teilweise an einem entsprechend gebogenen Bereich des Wadenteils entlang bewegbar sein.

Beispielsweise kann das Fussteil mit seinem gebogenen Bereich in den gebogenen Bereich des Wadenteils eingeschoben werden, wobei der gebogene Bereich des Wadenteils aussenseitig an der Stützschalenanordnung zu liegen kommt. Andererseits ist es auch denkbar, dass der gebogene Bereich des Wadenteils in den gebogenen Bereich des Fussteils verschoben wird, wobei der gebogene Bereich des Fussteils aussenseitig an der Stützschalenanordnung zu liegen kommt.

Die Führungsschiene der Feststelleinrichtung kann fersenseitig am Fussteil angeordnet, insbesondere einstückig mit dem Fussteil ausgebildet sein, oder die Führungsschiene der Feststelleinrichtung kann fersenseitig am Wadenteil angeordnet, insbesondere einstückig mit dem Wadenteil ausgebildet sein.

Das heisst, die Führungsschiene kann am Fussteil oder am Wadenteil angeordnet sein. Sämtliche Ausführungen, welche im Zusammenhang mit einer Führungsschiene am Fussteil gemacht werden, gelten daher analog auch für eine Führungsschiene am Wadenteil, und umgekehrt.

Die Führungsschiene kann am Fussteil angeordnet sein und das Wadenteil kann eine Ausbuchtung, Vertiefung oder Ausnehmung zur Aufnahme des Feststellelements aufweisen, so dass das Wadenteil zusammen mit dem Feststellelement entlang der Führungsschiene verschiebbar ist. Alternativ kann die Führungsschiene am Wadenteil angeordnet sein und das Fussteil kann eine Ausbuchtung, Vertiefung oder Ausnehmung zur Aufnahme des Feststellelements aufweisen, so dass das Fussteil zusammen mit dem Feststellelement entlang der Führungsschiene verschiebbar ist.

Diese Ausbuchtung, Vertiefung oder Ausnehmung bzw. Aussparung ist vorzugsweise im gebogenen Bereich des Wadenteils respektive im gebogenen Bereich des Fussteils ausgebildet und begrenzt einen Raum in welchen das Feststellelement passgenau aufgenommen werden kann. Bei der ersten Ausführungsform handelt es sich bevorzugt um eine Ausbuchtung oder Vertiefung, bei der zweiten Ausführungsform bevorzugt um eine Ausnehmung.

Die Führungsschiene kann aussenseitig am Fussteil angeordnet sein und das Wadenteil kann innenseitig eine Führungsnut zur geführten Aufnahme der Führungsschiene aufweisen.

Wird nun das Wadenteil über das Fussteil gestülpt, so wird die Führungsnut zunehmend über die Führungsschiene gefühl1. Anders gesagt, wird das Fussteil in das Wadenteil eingeschoben, so wird die Führungsschiene zunehmend in die Führungsnut im Wadenteil eingeführt. Analog kann die Führungsschiene auch am Wadenteil angeordnet sein und in eine Führungsnut am Fussteil geführt eingeführt werden. Die Führungsnut stellt in beiden Fällen sicher, dass kein seitliches Verkippen des Wadenteils beziehungsweise des Fussteils möglich ist.

Das Feststellelement kann insbesondere bei der ersten Ausführungsform, aber auch bei der zweiten Ausführungsform, zwischen dem Wadenteil und dem Fussteil angeordnet sein. Dabei wird das Feststellelement bevorzugt vom Federelement, welches insbesondere zwischen dem Wadenteil und dem Feststellelement oder dem Fussteil und dem Feststellelement angeordnet sein kann, mit einer Federkraft in Richtung des Fussteils oder des Wadenteils beaufschlagt.

Das Wadenteil kann über mindestens einen Schlitz verfügen, welcher sich zumindest teilweise in das Wadenteil hinein erstreckt, so dass eine flexible Anpassung des Wadenteils an die Wade eines Patienten ermöglicht ist. Dieser Schlitz oder mehrere solcher Schlitze können sich zumindest teilweise durch das Wadenteil hindurch erstrecken und dem Wadenteil dadurch eine gewisse Verformbarkeit oder Formelastizität verleihen. So kann aufgrund der Schlitze eine flexible Anpassung des Wadenteils an die Wade eines Patienten erfolgen, während gleichzeitig aufgrund einer hohen Eigensteifigkeit des Wadenteils die notwendige Formstabilität dennoch bereitgestellt wird.

Die Stützschalenanordnung kann weiter ein Sohlenteil umfassen, welches mit dem Fussteil verbindbar ist, wobei das Sohlenteil über eine Drucksensoreinrichtung zur Erfassung der Auftrittsbelastung des Sohlenteils durch einen Patienten verfügt, und wobei die Drucksensoreinrichtung mindestens einen Drucksensor aufweist, der zur Ausgabe eines Sensorsignals ausgebildet ist.

Das Sohlenteil kann dabei beispielsweise mittels einer Rastverbindung abnehmbar am Fussteil verbunden werden. So können Rastvorsprünge am Fussteil ausgebildet sein, welche in entsprechende Ausnehmungen am Sohlenteil einrasten, oder umgekehrt. Allerdings ist es auch denkbar, dass das Sohlenteil und das Fussteil einstückig ausgebildet sind. Bevorzugt ist im Fussteil jedoch eine der äusseren Form des Sohlenteils entsprechende Vertiefung vorgesehen, in welche das Sohlenteil einlegbar ist.

Diese Drucksensoreinrichtung kann zur Information des Patienten über seine Auftrittsbelastung dienen. So ist es insbesondere bei Verletzungen im Bereich des Bein- oder Fussskeletts oder der dortigen Bänder und Knorpel wichtig, dass der Patient im Rahmen der Rehabilitation beim Gehen oder Stehen eine gewisse maximale Auftrittsbelastung nicht überschreitet. Indem nun die Stützschalenanordnung über ein Sohlenteil mit einem oder mehreren Drucksensoren verfügt, kann die Auftrittsbelastung mittels der Drucksensoren erfasst und weiter verwertet werden.

Die Stützschalenanordnung kann weiter eine Auswerteeinrichtung, in welcher ein vorgegebener Maximalwert und/oder Maximalwertebereich der zulässigen Auftrittsbelastung speicherbar ist, und welche dazu ausgebildet ist, einen Sensorwert aus dem Sensorsignal des Drucksensors zu ermitteln und den ermittelten Sensorwert mit dem vorgegebenen Maximalwert und/oder dem Maximalwertebereich zu vergleichen, und eine Informationsübermittlungseinheit, welche dazu ausgebildet ist, bei einer Überschreitung des Maximalwerts und/oder des Maximalwertebereichs durch den ermittelten Sensorwert ein optisches und/oder akustisches Signal auszusenden, umfassen. Ein Schalter, insbesondere ein Druckknopf, kann vorgesehen sein, um bzgl. der Auftrittsbelastung einen Nullpunkt zu definieren. Bevorzugt ist die Auswerteeinrichtung dazu ausgebildet, diesen Nullpunkt im Hinblick auf die Auswertung zu speichern. Der Nullpunkt kann insbesondere der beim normalen aufrechten Stehen von dem oder den Drucksensoren gemessenen Belastung entsprechen.

Diese Elemente erlauben es zum Beispiel einem Arzt, einen Maximalwert oder Maximalwerte für eine zulässige Auftrittsbelastung in der Auswerteeinrichtung der Stützschalenanordnung zu programmieren. Tritt dann der Patient mit einer Auftrittsbelastung auf das Sohlenteil, welche diese vorgegebenen Maximalwerte übersteigt, so wird er zum Beispiel mittels einer aufleuchtenden Warnlampe und/oder eines erklingenden Alarmtones darüber informiert und kann seine Auftrittsbelastung umgehend entsprechend verringern. Indem eine Überbelastung vermieden wird, kann der Heilungsprozess beschleunigt werden, was unter anderem auch zu einer Kostensenkung im Gesundheitswesen führt.

Die Stützschalenanordnung kann weiter eine Energieversorgungseinrichtung zur Versorgung der Auswerteeinrichtung und/oder der Informationsübermittlungseinheit mit Energie sowie einen Arbeitsschalter umfassen, wobei der Arbeitsschalter dazu ausgebildet ist, die Energieversorgung im Falle einer Auftrittsbelastung einzuschalten und die Energieversorgung, falls während einer gewissen Zeit keine Auftrittsbelastung detektiert wird, auszuschalten. Indem eine Energieversorgung nur bei Gebrauch der Stützschalenanordnung erfolgt, wird die Lebensdauer der Energieversorgungseinrichtung verlängert.

Obwohl die Drucksensoreinrichtung, die Auswerteeinrichtung, die Informationsübermittlungseinheit und die Energieversorgungseinrichtung hier im Zusammenhang mit der Stützschalenanordnung beschrieben wurden, ist es genauso denkbar, diese Elemente beispielsweise in Form einer Sohleneinlage zum Beispiel für einen Gips oder einen orthopädischen Schuh vorzusehen.

Die Stützschalenanordnung weist ausserdem vorteilhaft ein Schienbeinteil auf, welches gemeinsam mit dem Wadenteil dazu ausgebildet ist, den Unterschenkel des Patienten zu umwinden bzw. zu umgeben, vorzugsweise vollständig zu umwinden bzw. zu umgeben. Bevorzugt sind das Schienbeinteil und das Wadenteil sogar dazu ausgebildet, den Unterschenkel derart zu umgeben, dass sie einander in den einander angrenzenden Bereichen überlappen. Vorzugsweise ist zudem ein Fussrückenteil vorhanden, welches gemeinsam mit dem Fussteil dazu ausgebildet ist, den Fuss des Patienten zu umwinden bzw. zu umgeben, vorzugsweise vollständig zu umwinden bzw. zu umgeben. Die Stützschalenanordnung weist dadurch eine gute Stabilität mit optimalem Kräftefluss auf.

Wie bereits erwähnt, müssen Stützschalenanordnungen eine für eine Stützung ausreichende Stabilität aufweisen, wozu deren Bestandteile wie das Fussteil oder das Wadenteil eine gewisse Eigensteifigkeit besitzen. Trotz anpassungsfähigen, flexiblen Stützvorrichtungen können daher dennoch für den Patienten unangenehme Druckstellen entstehen.

In einem zweiten Aspekt wird daher eine Einlage, insbesondere ein Innenschuh, für eine Stützschalenanordnung, insbesondere für eine Stützschalenanordnung wie oben beschrieben, angegeben, welche eine erste Kammer und eine zweite Kammer umfasst. Die Einlage weist zudem eine erste Ventileinrichtung auf, mittels welcher ein Fluid wie insbesondere Luft in die erste Kammer einfüllbar ist, um die Einlage in einem ersten Bereich zu versteifen. Die zweite Kammer beinhaltet eine Vielzahl von Formkörpern. Ausserdem weist die Einlage eine zweite Ventileinrichtung auf, mittels welcher ein Fluid aus der zweiten Kammer abführbar ist, um die Einlage in einem zweiten Bereich zu versteifen.

Eine mögliche Anwendung dieser Einlage kann darin bestehen, dass diese in einer Stiitzschalenanordnung eingesetzt wird und damit in Kontakt mit einem Patienten kommt. Dann wird die noch unbefüllte erste Kammer sukzessiv mit einem Fluid gefüllt, bis sie am zu stützenden Körperteil wie ein Polster anliegt, und die zweite Kammer wird evakuiert, wobei sich deren Formkörper konturnah an das zu stützende Körperteil anlegen. Über die erste Ventileinrichtung kann das Fluid jederzeit aus der ersten Kammer abgelassen beziehungsweise über die zweite Ventileinrichtung kann ein Fluid jederzeit in die zweite Kammer eingelassen werden, so dass die Einlage reversibel versteifbar ist. Die Einlage wird so optimal an die Konturen des zu stützenden Körperteils angepasst, wodurch Druckstellen vermieden werden und ein hoher Tragekomfort der Stützschalenanordnung ermöglicht wird.

Eine besonders einfache und platzsparende Ausführungsform ergibt sich, wenn die erste und die zweite Ventileinrichtung durch eine gemeinsame Pumpvorrichtung gebildet werden, welche von einem Pump- zu einem Saugmodus und umgekehrt umschaltbar ist. Die gemeinsame Pumpvorrichtung weist bevorzugt einen einzigen Blasebalg auf, welcher sowohl für die Pump- als auch für die Saugfunktion verwendbar ist.

Es gilt zu verstehen, dass diese Einlage jedoch nicht nur mit einer Stützschalenanordnung verwendet werden kann, wie sie oben beschrieben ist, sondern zum Beispiel auch in eine einstückige Stütze oder in einen Gips eingesetzt werden kann. Auch ist deren Verwendung nicht auf den medizinischen Bereich beschränkt. Beispielsweise kann die Einlage auch als Innenschuh für einen Sportschuh wie einen Skischuh oder einen Wanderschuh verwendet werden. Umgekehrt muss eine Stützschalenanordnung mit der erwähnten Feststelleinrichtung nicht unbedingt eine derartige Einlage aufweisen. Die erwähnte Einlage ist deshalb insbesondere auch unabhängig von der angegebenen Stützschalenanordnung verkauf- und vermarktbar.

Die Einlage kann weiter eine Halterung umfassen, in welcher eine Luftpumpe haltbar ist, wobei die Luftpumpe mit der ersten Ventileinrichtung zum Befüllen der ersten Kammer mit Luft verbindbar ist. Diese Halterung kann zum Beispiel die Form einer Seitentasche aufweisen, in welche die Luftpumpe eingesteckt wird und dadurch stets mit der Einlage mitgetragen wird. Dadurch wird einem Patienten ermöglicht, jederzeit eine Anpassung der Einlage vorzunehmen.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine perspektivische Ansicht einer Stützschalenanordnung gemäss einer ersten erfindungsgemässen Ausführungsform ohne Schienbein- und Fussrückenteil und ohne Befestigungsbänder, aber mit Sohlenteil, in einer ersten Beugeposition;
- Fig. 2: eine perspektivische Ansicht der Stützschalenanordnung gemäss Figur 1 mit Schienbein- und Fussrückenteil und mit Befestigungsbändern und Sohlenteil, in einer zweiten Beugeposition;
- Fig. 3: eine perspektivische teilweise Explosionsansicht der Stützschalenanordnung gemäss Figur 1 von schräg unten ohne Schienbein- und Fussrückenteil, ohne Sohlenteil und ohne Befestigungsbänder;
- Fig. 4: eine Detailansicht des Bereichs A in Figur 3;
- Fig. 5: eine perspektivische Ansicht einer Stützschalenanordnung gemäss einer zweiten erfindungsgemässen Ausführungsform mit Sohlenteil und einem Befestigungsband, aber ohne Schienbein- und Fussrückenteil;
- Fig. 6: eine weitere perspektivische Ansicht der Stützschalenanordnung gemäss Figur 5 ohne Befestigungsband, Schienbein- und Fussrückenteil und Sohlenteil,
- Fig. 7: eine teilweise Explosionsdarstellung der Stützschalenanordnung gemäss Figur 5 ohne Befestigungsband und Schienbein- und Fussrückenteil, aber mit Sohlenteil;
- Fig. 8: eine weitere teilweise Explosionsdarstellung der Stützschalenanordnung gemäss Figur 5 ohne Befestigungsband, Schienbein- und Fussrückenteil und Sohlenteil;
- Fig. 9: eine Detailansicht des Bereichs B in Figur 8;
- Fig. 10: eine weitere teilweise Explosionsdarstellung der Stützschalenanordnung gemäss Figur 5 ohne Befestigungsband, Schienbein- und Fussrückenteil und Sohlenteil;
- Fig. 11: eine weitere teilweise Explosionsdarstellung der Stützschalenanordnung gemäss Figur 5 ohne Befestigungsband, Schienbein- und Fussrückenteil und Sohlenteil;
- Fig. 12: eine perspektivische Ansicht der Stützschalenanordnung gemäss Figur 5 ohne Befestigungsband sowie Schienbein- und Fussrückenteil, aber mit unverbundenem Sohlenteil;
- Fig. 13: eine perspektivische Ansicht der Stützschalenanordnung gemäss Figur 5, wobei das Sohlenteil als Teilschnitt dargestellt ist;
- Fig. 14: eine schematische Seitenansicht des Sohlenteils gemäss Figur 13;
- Fig. 15: eine perspektivische Ansicht eines Innenschuhs mit einer ersten und zweiten Kammer für eine Stützschalenanordnung; sowie
- Fig. 16: eine schematische Schnittansicht der zweiten Kammer entlang der Ebene C-C in Figur 15 in einem mit Luft befüllten und in einem evakuierten Zustand.

### BESCHREIBUNG BEVORZUGTER AUSFÜHRUNGSFORMEN

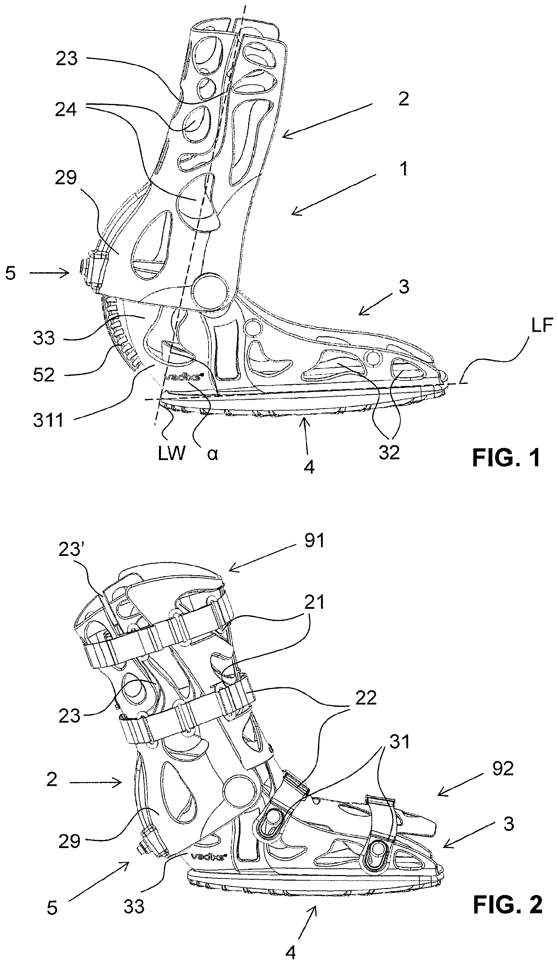

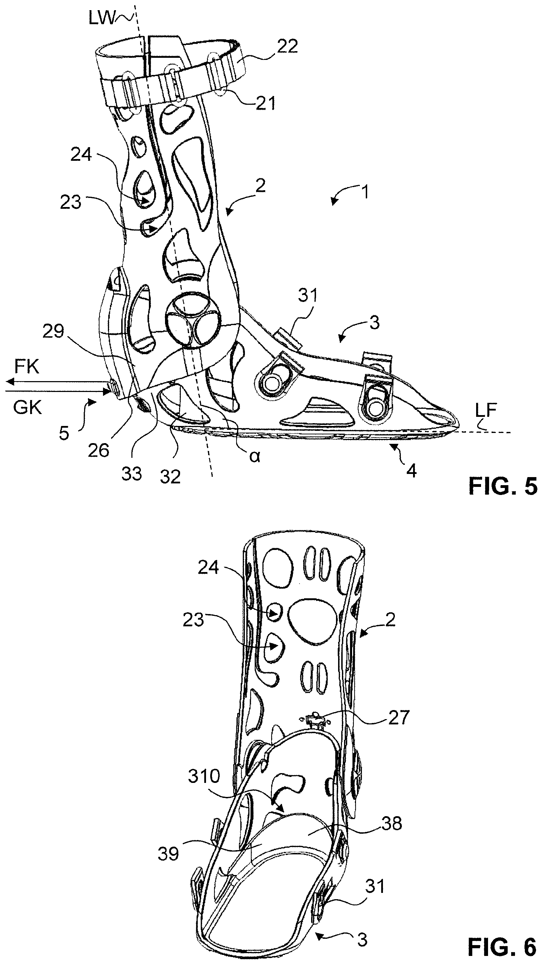

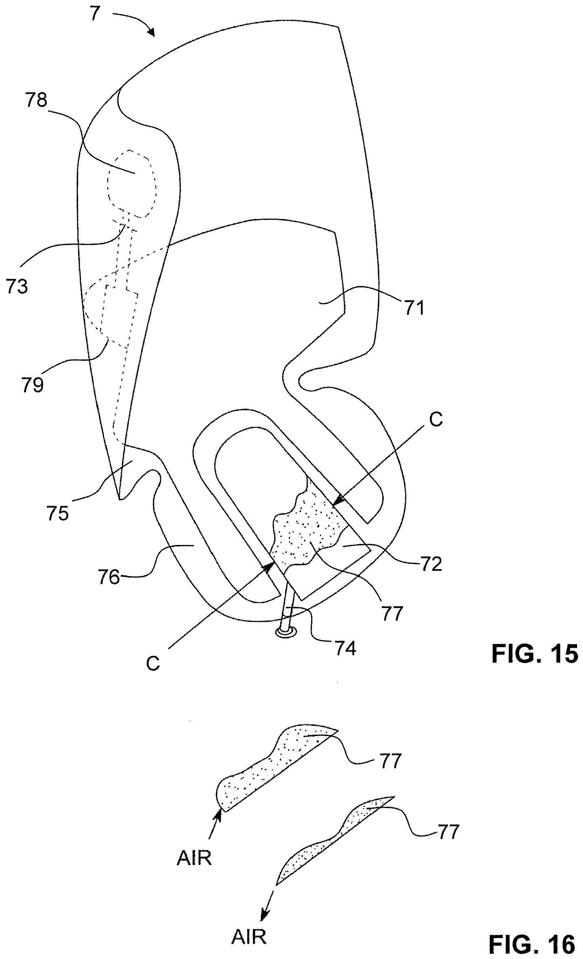

In den Figuren 1 bis 4 werden unterschiedliche Ansichten einer Stützschalenanordnung 1 gemäss einer ersten Ausführungsform umfassend ein Wadenteil 2, ein Fussteil 3, ein Sohlenteil 4 sowie eine Feststelleinrichtung 5 dargestellt. Bei der Stützschalenanordnung 1 gemäss dieser in den Figuren 1 bis 4 gezeigten ersten Ausführungsform dient ein Federelement 53 dazu, das Verändern des Beugewinkels zwischen dem Wadenteil 2 und dem Fussteil 3 zu erleichtern. Eine Stützschalenanordnung 1 gemäss einer zweiten Ausführungsform ist in den Figuren 5 bis 14 dargestellt, wobei diese zweite Ausführungsform im Gegensatz zur ersten Ausführungsform ein Federelement 53 aufweist, welches dazu dient, das Verändern des Beugewinkels zwischen dem Wadenteil 2 und dem Fussteil 3 zu erschweren. Ein Innenschuh 7, welcher als Einlage für eine Stützschalenanordnung 1 gemäss der ersten oder auch zweiten Ausführungsform verwendet werden kann, ist in Figur 15 dargestellt.

Im Folgenden sind bei unterschiedlichen Ausführungsformen Merkmale, welche dieselbe oder eine ähnliche Ausgestaltung und/oder dieselbe oder eine ähnliche technische Wirkung aufweisen, jeweils mit denselben Bezugszeichen versehen.

Wie aus den Figuren 1 bis 4 hervorgeht, weist die Stützschalenanordnung 1 gemäss der ersten Ausführungsform ein Wadenteil 2 zur Aufnahme eines Unterschenkels sowie ein Fussteil 3 auf, welches fersenseitig mit dem Wadenteil 2 verbindbar ist. Zur kraftschlüssigen Anordnung der Stützschalenanordnung 1 am Unterschenkel eines Patienten sind am Wadenteil 2 sowie am Fussteil 3 Gurtlaschen 21, 31 vorgesehen, an welchen jeweils ein Befestigungsgurt 22 oder Befestigungsband befestigt werden kann. Beispielsweise kann ein längenverstellbares, sogenanntes Velcro-Band verwendet werden, welches durch die Gurtlaschen 21 des Wadenteils 2 geführt und um die Wade des Patienten geschlungen wird und so den Unterschenkel eines Patienten an der Stützschalenanordnung befestigt. Zwischen den Befestigungsgurten 22 und dem Unterschenkel bzw. dem Fuss können zur weiteren Stabilisierung ein Schienbeinteil 91 sowie ein Fussrückenteil 92 vorgesehen sein. Das Schienbeinteil 91 ist gemeinsam mit dem Wadenteil 2 dazu ausgebildet, den Unterschenkel des Patienten vorzugsweise vollständig umlaufend zu umgeben bzw. zu umwinden. Dank dieser Umwindung des Unterschenkels in Kombination mit der durch die Feststelleinrichtung bewirkten Fixierung des Fusses relativ zum Unterschenkel wird eine optimale Stabilisierung des Fussgelenkes erreicht. Die beim Gehen auftretenden Kräfte werden dabei durch die Stützschalenanordnung 1 aufgenommen und können aufgrund der erwähnten Umwindung des Unterschenkels gut abgeleitet werden.

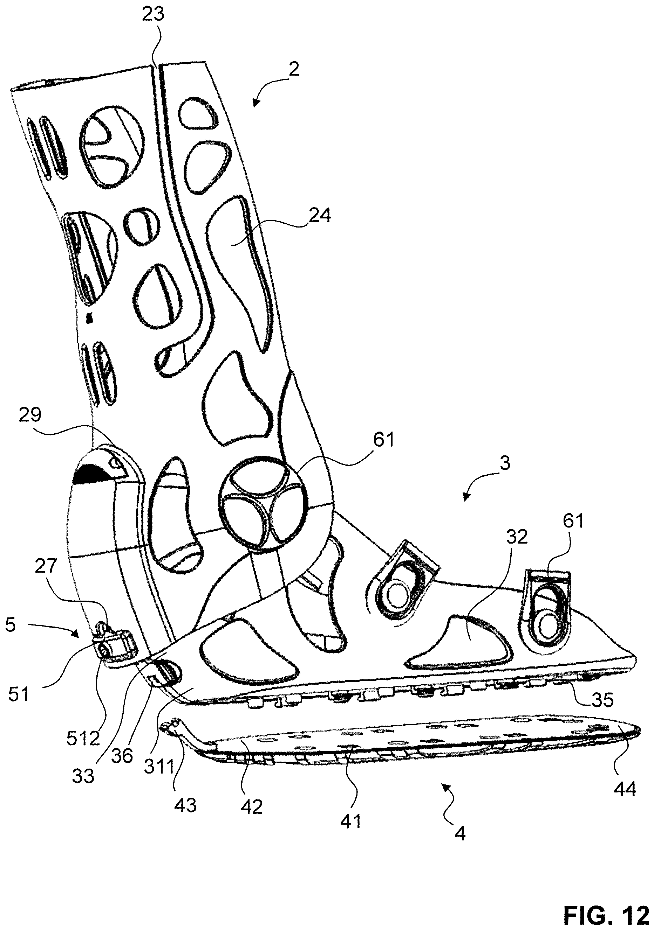

Im vorliegenden Fall ist das Wadenteil 2 einstückig ausgebildet und verfügt beidseitig jeweils über einen Schlitz 23, 23', welcher sich von der Einstiegsseite des Wadenteils 2 gesehen zumindest teilweise in das Wadenteil 2 hinein erstreckt. Diese Schlitze 23, 23' verleihen dem Wadenteil 2 eine gewisse Verformbarkeit respektive Formelastizität und ermöglichen dadurch eine flexible Anpassung des Wadenteils 2 an die Wade des Patienten. Sowohl das Wadenteil 2 wie auch das Fussteil 3 verfügen über weitere Öffnungen 24, 32, welche einer Belüftungsfunktion dienen. Auf das Sohlenteil 4 wird im Zusammenhang mit den Figuren 12 bis 14 genauer eingegangen.

In den hier gezeigten Figuren ist das Fussteil 3 fersenseitig gebogen und wird bei einem Verschieben des Wadenteils 2 in Richtung des Fussteils 3 mit diesem gebogenen Bereich 311 zumindest teilweise in das Wadenteil 2 eingeführt. Das Wadenteil 2 weist dazu fersenseitig ebenfalls einen entsprechend gebogenen Bereich 29 auf, welcher sich beim Einschieben des Fussteils 3 in das Wadenteil 2 über das gebogene Fussteil stülpt.

Weiter ist eine Feststelleinrichtung 5 vorgesehen, welche ein Feststellelement 51, eine Führungsschiene 52 sowie ein Federelement 53 umfasst. In dem hier gezeigten Beispiel ist die Führungsschiene 52 aussenseitig in einem proximalen Bereich 33 des Fussteils 3, also fersenseitig, am Fussteil 3 ausgebildet. Die Führungsschiene 52 erstreckt sich dabei im Wesentlichen entlang des gesamten gebogenen Bereichs 311 des Fussteils 3 sowie in Richtung des Wadenteils 2 etwas über das Fussteil 3 hinaus.

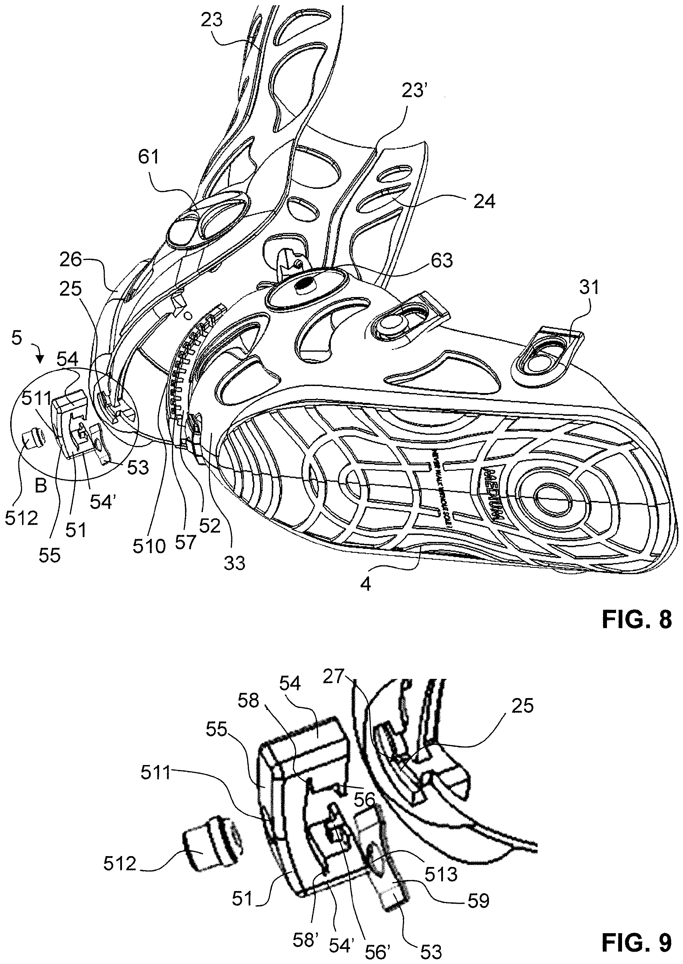

Die Führungsschiene 52 wird beim Zusammenfügen des Wadenteils 2 mit dem Fussteil 3 zunächst in einer Führungsnut 25, welche vorteilhaft innenseitig im gebogenen Bereich 29 des Wadenteils 2 ausgebildet ist, aufgenommen. Die Führungsnut 25 ist bei der in den Figuren 1 bis 4 gezeigten ersten Ausführungsform nicht erkennbar, aber vorteilhaft vorhanden. In Bezug auf die zweite Ausführungsform ist die Führungsnut 25 in den Figuren 8 und 9 gut erkennbar. Beim weiteren Zusammenfügen wird der restliche Bereich der Führungsschiene 52 zunehmend in die Führungsnut 25 eingeschoben. Steht das Wadenteil 2 im Wesentlichen senkrecht auf dem Fussteil 3, so ist die Führungsschiene 52 praktisch vollständig in der Führungsnut 25 aufgenommen.

Wie in den Figuren 8 und 9 ersichtlich ist, ist die Führungsnut 25 als eine T-förmige Aussparung ausgebildet, in welche die komplementär dazu ausgebildete Führungsschiene 52 in Form einer T-förmigen Erhebung nahezu spielfrei aufgenommen wird. Dank dieser geführten Bewegung kann ein seitliches Verkippen des Wadenteils 2 gegenüber dem Fussteil 3 vermieden werden. Je nach Verschiebung des Wadenteils 2 entlang der Führungsschiene 52 des Fussteils 3 wird ein bestimmter Beugewinkel α zwischen einer Hauptlängsachse LW des Wadenteils 2 und einer Hauptlängsachse LF des Fussteils 3 eingestellt. Um das Wadenteil 2 in einer gewünschten Position beziehungsweise in einem bestimmten Beugewinkel α zu fixieren, ist die Feststelleinrichtung 5, insbesondere das Feststellelement 51 mit dem Federelement 53, vorgesehen.

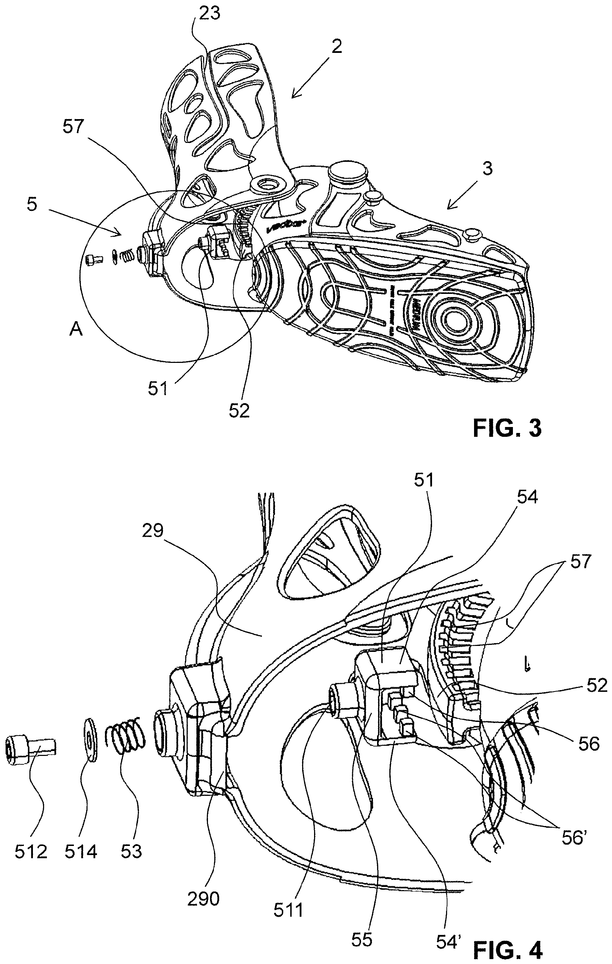

Im gebogenen Bereich 29 des Wadenteils 2 ist eine sich in Richtung vom Fussteil 3 weg und nach aussen hin erstreckende Ausbuchtung 290 ausgebildet, welche zur passgenauen Aufnahme des Feststellelements 51 dient (siehe Figuren 3 und 4). Das Feststellelement 51 ist somit zwischen dem Wadenteil 2 und dem Fussteil 3 angeordnet. Das Feststellelement 51 ist als Ganzes im Wesentlichen U-förmig ausgestaltet mit zwei Schenkeln 54, 54' sowie einen diese zwei Schenkel 54, 54' verbindenden Steg 55. Im Steg 55 ist eine zentrale Durchgangsöffnung 511 ausgebildet, innerhalb welcher ein Innengewinde ausgebildet ist. Beim Verstellen des Beugewinkels α zwischen dem Wadenteil 2 und dem Fussteil 3 wird das in der Ausbuchtung 290 aufgenommene Feststellelement 51 zusammen mit dem Wadenteil 2 entlang der Führungsschiene 52 verfahren.

Es ist jedoch genauso denkbar, dass die Führungsschiene 52 nicht am Fussteil 3 sondern am Wadenteil 2 angeordnet ist, und dass das Feststellelement 51 entsprechend in einer Vertiefung im proximalen Bereich 33 des Fussteils 3 aufnehmbar ist. Sämtliche Ausführungen betreffend eine Führungsschiene 52, welche am Fussteil 3 angeordnet ist beziehungsweise betreffend ein Feststellelement 51, welches im Wadenteil 2 aufnehmbar ist, gelten daher analog auch für eine Führungsschiene, welche am Wadenteil 2 angeordnet ist beziehungsweise für ein Feststellelement, welches im Fussteil 3 aufnehmbar ist. Das Feststellelement 51 könnte zudem grundsätzlich auch mit dem Wadenteil 2 (bzw. dem Fersenteil 3) verbunden oder sogar einstückig an diesem angebracht sein. Dies wäre beispielsweise dann möglich, wenn der gebogene Bereich 29 des Wadenteils 2 eine gewisse Flexibilität aufweist, welche eine Bewegung des mit diesem einstückig ausgebildeten Feststellelements 51 zur Führungsschiene 52 hin und von dieser weg ermöglicht.

Wie weiter aus den Figuren 3 und 4 hervorgeht, weisen die beiden Schenkel 54, 54' des Feststellelements 51 im Bereich ihrer freien Enden jeweils Einrastelemente in Form von jeweils drei Rastnasen 56, 56' auf, auf welche später genauer eingegangen wird. Gleiches gilt für eine entsprechend dazu ausgebildete Einraststruktur, beispielsweise in Form von Rastkerben 57, welche an der Führungsschiene 52 vorgesehen sind.

Das Feststellelement 51 hintergreift mit seinen Rastnasen 56, 56' die im Querschnitt T-förmige Führungsschiene 52 von beiden gegenüberliegenden Seiten her. Dabei greifen die Rastnasen 56, 56' in einer Fixierposition in die Rastkerben 57 der Führungsschiene 52 ein, wodurch ein Bewegen des Feststellelements 51 entlang der Führungsschiene 52 verhindert wird. Indem das Feststellelement 51 zum Fussteil 3 hin gedrückt wird, werden die Rastnasen 56, 56' ausser Eingriff mit den Rastkerben 57 gebracht, so dass das Feststellelement 51 eine Verschiebeposition einnimmt. In der Verschiebeposition ist das Feststellelement 51 frei entlang der Führungsschiene 52 bewegbar, so dass der Beugewinkel α zwischen Wadenteil 2 und Fussteil 3 einstellbar ist bzw. dem Patienten beim Gehen eine gewisse Bewegungsfreiheit gewährt wird.

Durch eine in der Ausbuchtung 290 zentral vorgesehene Durchgangsöffnung ragt ein Fixierelement 512 in Form einer Fixierschraube hindurch. Mit seinem Aussengewinde ist die Fixierschraube 512 in das am Feststellelement 51 ausgebildete Innengewinde der Durchgangsöffnung 511 eingeschraubt. Via eine Unterlagscheibe 514 liegt die Fixierschraube 512 zudem mit ihrem Schraubenkopf im Bereich der Ausbuchtung 290 an der Aussenseite des Wadenteils 2 an. Zwischen dem Feststellelement 51 und der Unterlagscheibe 514 ist ein Federelement 53 in Form einer Spiralfeder angeordnet, welche von der Fixierschraube 512 durchragt wird. Das Federelement 53 beaufschlagt das Feststellelement 51 mit einer zum Fussteil 3 hin gerichteten Kraft und hält das Feststellelement 51 somit, wenn die Fixierschraube 512 nur wenig in das Innengewinde der Durchgangsöffnung 511 eingeschraubt ist, in seiner Verschiebeposition, das heisst ausser Eingriff mit den Rastkerben 57 der Führungsschiene 52. Durch Einschrauben des Fixierelements 512 in das Innengewinde der Durchgangsöffnung 511 hinein kann das Feststellelement 51 in seine Fixierposition gebracht werden. Das Feststellelement 51 wird dann entgegen die vom Federelement 53 bewirkte Federkraft in Richtung zum Wadenteil 2 hin gezogen, wodurch die Rastnasen 56, 56' in Eingriff mit den Rastkerben 57 der Führungsschiene 52 gebracht werden.

Obwohl hier nicht dargestellt, ist es ebenfalls denkbar, die Führungsschiene 52 sowie das Feststellelement 51 ohne jegliche Raststrukturen vorzusehen und stattdessen einzig jeweils eine Anschlagfläche am Feststellelement sowie an der Führungsschiene vorzusehen, welche in der Fixierposition aneinander anschlagen. Dadurch wäre ein Verschieben des Wadenteils 2 entlang der Führungsschiene 52 des Fussteils 3 aufgrund der durch das Fixierelement 512 bewirkten Reibungskraft zumindest erschwert. Das Feststellelement und die Führungsschiene können an den entsprechenden Stellen zum Beispiel eine reibungserhöhende Beschichtung aufweisen.

Die in den Figuren 5 bis 14 dargestellte zweite Ausführungsfonn einer Stützschalenanordnung 1 ist mit einigen Ausnahmen, auf die anschliessend näher eingegangen wird, ähnlich, bevorzugt identisch, ausgestaltet wie die in den Figuren 1 bis 4 gezeigte erste Ausführungsform. Es werden im Folgenden in Bezug auf die zweite Ausführungsform somit vornehmlich die Unterschiede zur ersten Ausführungsform erläutert.

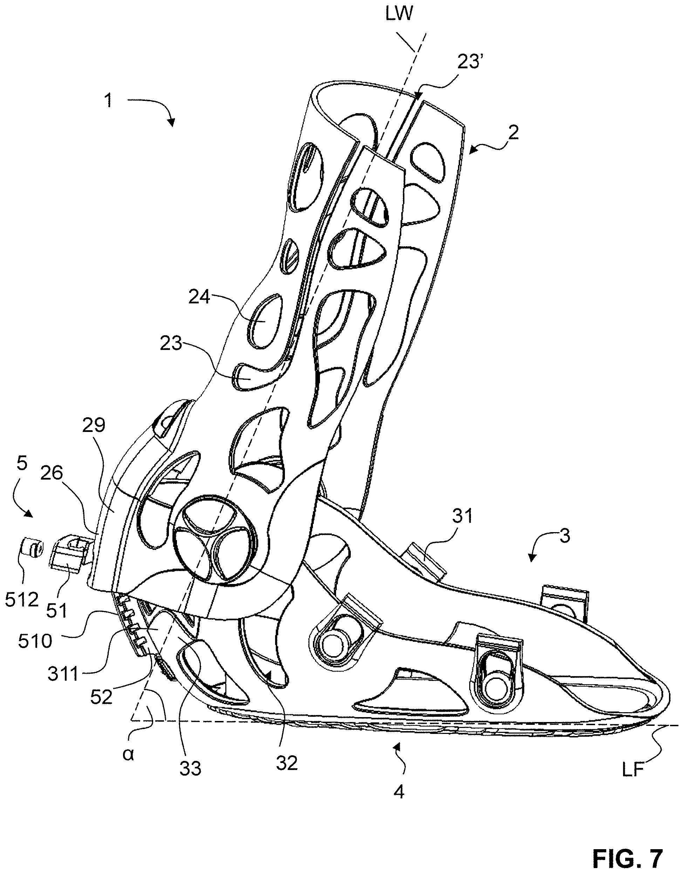

In den Figuren 7 bis 9 ist ersichtlich, dass das Feststellelement 51 ebenso wie bei der ersten Ausführungsform eine im Wesentlichen U-förmige Außenform aufweist und über zwei Schenkel 54, 54' sowie einen diese zwei Schenkel 54, 54' verbindenden Steg 55 verfügt. Das Wadenteil 2 wiederum verfügt im Gegensatz zur ersten Ausführungsform im proximalen Bereich 26 an einem unteren Ende jedoch über eine Ausnehmung 27, welche sich vollständig durch das Wadenteil 2 sowie die dort ausgebildete Führungsnut 25 hindurch erstreckt und dazu ausgebildet ist, das Feststellelement 51 im Wesentlichen passgenau aufzunehmen. Dadurch kann das in der Ausnehmung 27 aufgenommene Feststellelement 51 von einem Anwender, wie zum Beispiel einem Arzt, gemeinsam mit dem Wadenteil 3 entlang der Führungsschiene 52 verschoben werden.

Im Bereich des Stegs 55 verfügen die beiden Schenkel 54, 54' der zweiten Ausführungsform jeweils über einen Einschnitt 58, 58', in welche das Federelement 53, hier in Form einer Blattfeder, eingesetzt werden kann. Die Blattfeder 53 erstreckt sich im eingesetzten Zustand im Wesentlichen entlang der gesamten Länge des Stegs 55 und weist eine Krümmung 59 auf, welche zur Führungsschiene 52 hin gekrümmt ist (die tatsächliche Krümmung ist also bevorzugt entgegengesetzt zu der in den Figuren 8 und 9 falsch dargestellten Krümmung ausgerichtet). Diese Krümmung 59 erzeugt eine Federkraft FK, welche auf das Feststellelement 51 wirkt und dazu führt, dass das Feststellelement 51 zwar zwischen einer Verschiebeposition und einer Fixierposition bewegbar ist, jedoch in Richtung zur Fixierposition hin gedrückt wird. In der Fixierposition wirkt die Federkraft FK der Blattfeder 53 derart auf das Feststellelement 51, dass ein Verschieben des Feststellelements 51, und dadurch des Wadenteils 2, entlang der Führungsschiene 52 erschwert oder sogar verunmöglicht ist. Somit ist das Wadenteil 2 mittels des Feststellelements 51 an der Führungsschiene 52 vorfixiert. Diese Fixierposition kommt dabei deshalb zu Stande, weil die Krümmung 59 der Blattfeder 53 auf einer Anlagefläche 510 der Führungsschiene 52 zu liegen kommt und bewirkt, dass das Feststellelement 51 aufgrund der Federkraft FK von der Anlagefläche 510 nach aussen hin weg gedrückt wird. Dabei kommen die Rastnasen 56, 56' des Feststellelements 51 mit den Rastkerben 57 der Führungsschiene 52 in Eingriff und ein Verschieben des Wadenteils 2 entlang der Führungsschiene 52 ist nicht mehr möglich.

Um das Feststellelement 51 in die Verschiebeposition zu überführen, in welcher ein Verschieben des Feststellelements 51, und dadurch des Wadenteils 2, möglich ist, muss eine der Federkraft FK entgegengesetzte Kraft GK aufgewendet werden. Das heisst, wird eine entsprechende Gegenkraft GK auf das Federelement 53 ausgeübt, in anderen Worten, wird das Feststellelement 51 von aussen her durch die Ausnehmung 27 hindurch in Richtung der Führungsschiene 52 gedrückt, so wird der Eingriff zwischen den Rastnasen 56, 56' des Feststellelements 51 und den Rastkerben 57 der Führungsschiene 52 gelöst. Das Feststellelement 51, und somit auch das Wadenteil 2, kann dann entlang der Führungsschiene 52 bewegt werden. Entscheidend ist hierbei auch, dass diese Gegenkraft GK nicht nur entgegengesetzt zur Federkraft FK des Federelements 53 ist, sondern auch eine Stärke aufweist, welche gleich gross oder grösser als die Stärke der Federkraft FK ist.

Wie ebenfalls aus den Figuren 8 und 9 hervorgeht, kann der Steg 55 des Feststellelements 51 über eine Durchgangsöffnung 511 verfügen, durch welche ein Fixierelement 512 zur zusätzlichen Sicherung beziehungsweise Fixierung des Feststellelements 51 an der Führungsschiene 52 in der Fixierposition eingeführt werden kann. Insbesondere ist es denkbar, die Durchgangsöffnung 511 mit einem Innengewinde zu versehen und als Fixierelement 512 eine Fixierschraube mit einem Aussengewinde zu verwenden, welche dann in die Durchgangsöffnung 511 hineingeschraubt werden kann. Diese Fixierschraube 512 wird dabei solange in Richtung der Führungsschiene 52 geschraubt, bis die Fixierschraube 512 auf der Anlagefläche 510 der Führungsschiene 52 auftrifft. Im vorliegenden Fall weist das Federelement 53 deshalb ebenfalls eine Durchgangsöffnung 513 auf, durch welche die Fixierschraube 512 hindurchgeschraubt wird. Es ist jedoch auch denkbar, keine solche Durchgangsöffnung 513 am Federelement 53 vorzusehen. Dann würde im eingeschraubten Zustand nicht die Fixierschraube 512 direkt auf der Anlagefläche 510 der Führungsschiene 52 auftreffen, sondern die Fixierschraube 512 würde das Federelement 53 auf die Anlagefläche 510 pressen. In beiden Fällen wird dabei von der Fixierschraube 512 eine Kraft auf das Feststellelement 51 erzeugt, welche das Feststellelement 51 analog zum Federelement 53 von der Führungsschiene 510 nach aussen weg drückt, wodurch die Rastnasen 56, 56' des Feststellelements 51 in einen maximalen Eingriff mit den Rastkerben 57 der Führungsschiene 52 gebracht werden respektive wodurch ein Anschlag zwischen der Anschlagsfläche des Feststellelements und der Führungsschiene 52 entsteht. Dadurch ist ein Verschieben des Feststellelements 51 entlang der Führungsschiene 52 verunmöglicht. Diese zusätzlich zur Federkraft FK wirkende Fixierkraft verkleinert das Risiko einer ungewollten Verschiebung des Wadenteils 2 entlang der Führungsschiene 52 in der Fixierposition, und somit das ungewollte Verstellen eines zuvor optimal eingestellten Beugewinkels α zwischen dem Fussteil 3 und dem Wadenteil 2, wodurch die Tragsicherheit der Stützschalenanordnung 1 erhöht wird.

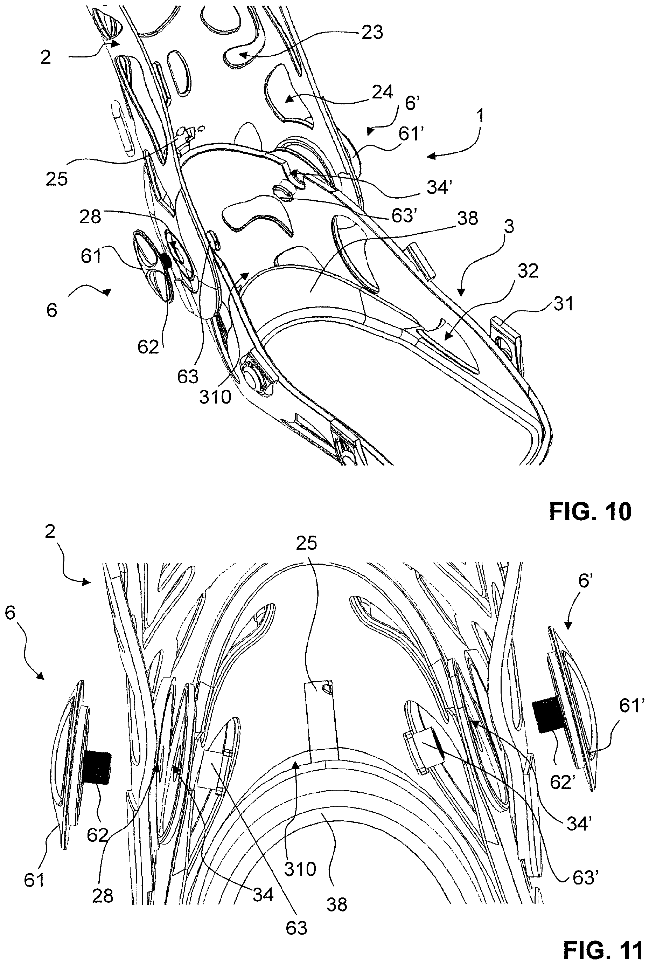

Insbesondere aus den Figuren 10 und 11 ist ersichtlich, dass die Stützschalenanordnung 1 gemäss der zweiten Ausführungsform weiter eine Gelenkeinrichtung 6, 6' zur gelenkigen Verbindung zwischen dem Wadenteil 2 und dem Fussteil 3 umfasst. Eine derartige Gelenkeinrichtung weist auch die in den Figuren 1 bis 4 gezeigte erste Ausführungsform auf. Diese Gelenkeinrichtung 6, 6' ist beidseitig seitlich an der Stützschalenanordnung 1 angeordnet und umfasst jeweils einen Drehknopf 61, 61' mit einer Gelenkstange 62, 62' sowie einen Zapfen 63, 63'. Sowohl das Wadenteil 2 als auch das Fussteil 3 verfügen beidseitig jeweils über eine Öffnung 28, 28', 34, 34', durch welche sich die Gelenkstange 62, 62' des Drehknopfs 61, 61' von ausserhalb der Stützschalenanordnung 1 ins Innere der Stützschalenanordnung 1 hinein erstreckt und dort drehfest im Zapfen 63, 63` aufgenommen wird. Die Gelenkstange 62, 62' kann dazu beispielsweise über ein Aussengewinde verfügen, welches in ein entsprechendes im Zapfen 63, 63' ausgebildetes Innengewinde eingeschraubt wird. Beim Tragen der Stützschalenanordnung 1 durch einen Patienten sind die Öffnungen 28, 28', 34, 34' leicht versetzt hinter der Fussgelenkachse des Patienten angeordnet.

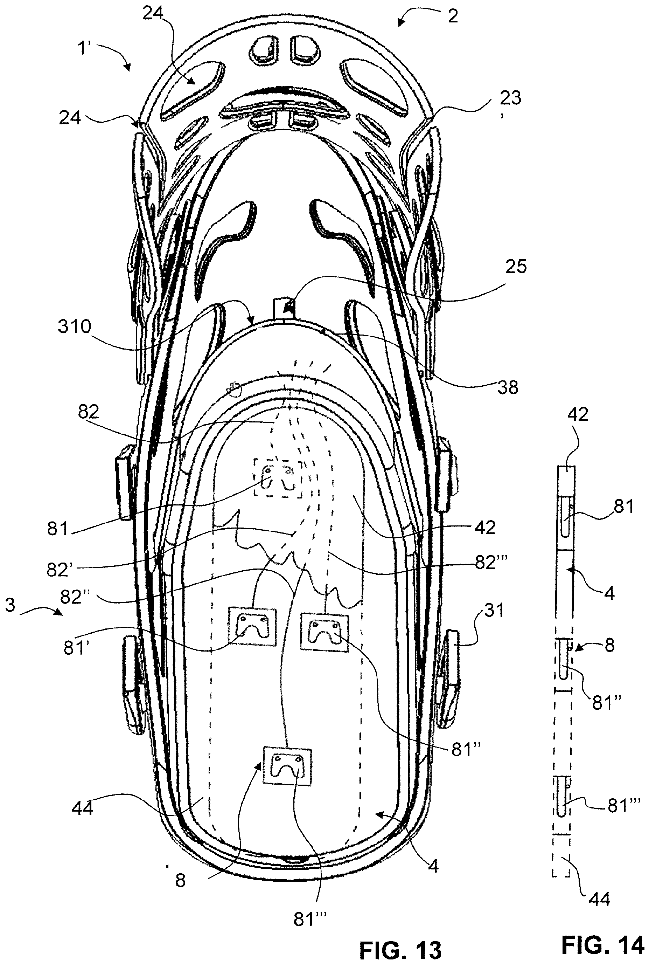

Wie eingangs erwähnt, weist die Stützschalenanordnung 1 sowohl der ersten als auch der zweiten Ausführungsform weiter ein Sohlenteil 4 auf, welches mit dem Fussteil 3 verbindbar ist. Insbesondere aus Figur 12 ist ersichtlich, dass das Sohlenteil 4 abnehmbar, beispielsweise über eine Schnapp- oder Rastverbindung, am Fussteil 3 befestigt werden kann. Dazu kann das Fussteil 3 über Vorsprünge 35 verfügen, welche in entsprechende Ausnehmungen 41 im Sohlenteil 4 einschnappen beziehungsweise einrasten oder umgekehrt. Zur stabileren Verbindung des Sohlenteils 4 mit dem Fussteil 3 kann das Sohlenteil 4 zusätzlich in einem proximalen Bereich 42 über eine verlängerte Verbindungsstruktur 43 verfügen, welche mit einer entsprechend dazu ausgebildeten Verbindungsstruktur 36 am proximalen Bereich 33 des Fussteils 3 verbunden werden kann. Auch diese Verbindung kann eine Schnapp- oder Rastverbindung sein. Das heisst, das Sohlenteil 4 kann durch "Aufklicken" auf sehr einfache Weise am Fussteil 3 befestigt werden. Anstelle eines abnehmbaren Sohlenteils 4 können das Fussteil 3 und das Sohlenteil 4 jedoch auch untrennbar miteinander verbunden beziehungsweise einstückig ausgebildet sein. Das Sohlenteil 4 kann optional eine Drucksensoreinrichtung zur Erfassung der Auftrittsbelastung des Sohlenteils 4 aufweisen, wie es nachfolgend in Bezug auf die in den Figuren 13 und 14 gezeigte zweite Ausführungsform erläutert wird.

In den Figuren 13 und 14 wird somit in Bezug auf die zweite Ausführungsform der Stützschalenanordnung 1 ein Sohlenteil 4 erläutert, welches eine Drucksensoreinrichtung 8 zur Erfassung der Auftrittsbelastung des Sohlenteils 4 aufweist. Selbstverständlich kann auch die erste, in den Figuren 1 bis 4 gezeigte Ausführungsform ebenfalls ein derartiges Sohlenteil 4 mit einer Drucksensoreinrichtung aufweisen.

In dem in Figur 13 gezeigten Beispiel weist die Drucksensoreinrichtung 8 vier Drucksensoren 81, 81', 81", 81''' auf, welche jeweils zur Ausgabe eines Sensorsignals ausgebildet sind. Hier befindet sich ein Drucksensor 81 im proximalen Bereich 42, ein Drucksensor 81''' im distalen Bereich 44 sowie zwei Drucksensoren 81', 81" nebeneinander in einem dazwischen angeordneten zentralen Bereich des Sohlenteils 4. Während die proximal und distal angeordneten Sensoren 81, 81''' vor allem die Auftrittsbelastung durch die Ferse beziehungsweise den Fussballen messen, dienen die zentral angeordneten Sensoren 81', 81" dazu, ein seitliches Verkippen des Fusses zu erfassen. Damit die Sensorsignale der Drucksensoren 81, 81', 81", 81''' ausgewertet werden können, ist jeder Sensor über eine eigene Leitung 82, 82', 82", 82''' mit einer (nicht dargestellten) Auswerteeinrichtung verbunden. Über diese Leitungen 82, 82', 82", 82''' werden die erfassten Sensorsignale an die Auswerteeinrichtung übermittelt und dort in Sensorwerte ausgewertet, insbesondere summiert, wodurch ein Vergleich mit vorgegebenen Referenzwerten durch die Auswerteeinrichtung ermöglicht wird. Diese Referenzwerte können einem vorgegebenen Maximalwert oder Maximalwertebereich der zulässigen Auftrittsbelastung entsprechen, welche in der Auswerteeinrichtung gespeichert sind und mit den Sensorwerten verglichen werden. Weiter kann eine (ebenfalls nicht dargestellte) Informationsübermittlungseinheit vorgesehen sein, welche dazu ausgebildet ist, bei einer Überschreitung des Maximalwerts respektive des Maximalwertebereichs durch die Sensorwerte ein optisches und/oder akustisches Signal auszusenden. Zum Beispiel kann das optische Signal ein Aufleuchten einer mit der Auswerteeinheit verbundenen Kontrolllampe sein und das akustische Signal kann einem Pfeifton aus einem mit der Auswerteeinheit verbundenen Lautsprecher entsprechen. Auch ist es denkbar, Anzeigemittel zur Anzeige der ermittelten Sensorwerte, z.B. einen Display, oder eine Energieversorgungseinrichtung, z.B. in Form einer Batterie, zur Versorgung der Auswerteeinrichtung und/oder der Informationsübermittlungseinheit mit Energie vorzusehen. Eine Möglichkeit besteht zum Beispiel darin, die Drucksensoren 81, 81', 81 ", 81''' in einer Rüstung des Sohlenteils 4 einzubetten und jeweils über die elektrische Leitung 82, 82', 82", 82''' mit einer elektronischen Schaltung zu verbinden. Die elektronische Schaltung kann eine Batterie, z.B. eine CR2032 Knopfzelle, einen Druckknopf zur Einstellung des Maximalwerts der Auftrittsbelastung, z.B. 5, 10, 15, 20, 25, 30, 35, 40 kg, eine LED Lampe, welche eine Bestätigung der Auftrittsbelastung durch Blinken signalisiert, sowie einen Reset in Form einer Kalibrierungstaste aufweisen. Weiter können ein Lautsprecher, welcher bei Überschreitung des eingestellten Maximalwerts ein akustisches Signal abgibt sowie ein Display zur Anzeige der Auftrittsbelastung, jeweils ebenfalls über weitere elektronische Leitungen, mit der elektronischen Schaltung verbunden sein. Auch ist es denkbar, einen Arbeitsschalter an der elektronischen Schaltung vorzusehen, welcher dazu ausgebildet ist, die Energieversorgung im Falle einer Auftrittsbelastung einzuschalten und die Energieversorgung im Falle keiner Auftrittsbelastung auszuschalten. Das heisst, dieser Schalter erfüllt gewissermassen eine Weckfunktion, wobei eine Auftrittsbelastung nur dann ermittelt wird, wenn ein Patient die Stützanordnung 1 trägt und auf das Sohlenteil 4 drauftritt. Damit diese Elemente einen Patienten beim Tragen der Stützschalenanordnung 1 nicht beeinträchtigen, ist in der Stützschalenanordnung 1 eine Aufnahme 310 vorgesehen, in welchen die Auswerteeinrichtung und/oder die Informationsübermittlungseinheit und/oder die Anzeigemittel und/oder die Energieversorgungseinrichtung aufgenommen werden können. Wie insbesondere in den Figuren 6, 10 und 13 ersichtlich ist, kann das Fussteil 3 zu diesem Zweck fersenseitig über einen Fersenriemen 38 verfügen, welcher beabstandet vom proximalen Ende 37 des Fussteils 3 im Fussteil 3 angeordnet ist und in distaler Richtung einen Anschlag 39 für die Ferse eines Patienten bildet. In proximaler Richtung wird so eine Aufnahme in Form eines Zwischenraums oder Spalts 310 gebildet, in welchen die mit den Sensoren 81, 81', 81", 81''' verbundenen Elemente aufgenommen und darin verstaut werden können.

In Figur 15 ist eine Einlage 7 in Form eines Innenschuhs für eine der Stützschalenanordnungen 1, wie sie oben beschrieben wurden, dargestellt. Diese Einlage 7 dient einer prä- oder postoperativen oder posttraumatischen Ruhigstellung sowie Stabilisierung z.B. bei Fuß- oder Knöchelfrakturen, Unterschenkelfrakturen, Verstauchungen, Achillessehnenentzündung, Weichteilverletzungen, Athrodesen, Cacaneusfrakturen, Metatarsalfrakturen, et cetera. Dabei handelt es sich um eine für den Patienten bequeme, leicht anzupassende Einlage 7 vorzugsweise aus einem Textil, welche eine Behandlungsalternative zu Gipsverbänden darstellt. Dazu umfasst die Einlage 7 mindestens eine erste Kammer 71 mit einer ersten Ventileinrichtung 73 und eine zweite Kammer 72 mit einer zweiten Ventileinrichtung 74. Die erste Kammer 71 befindet sich in einem fersenseitigen bzw. proximalen Bereich 75 der Einlage 7 und erstreckt sich seitlich zumindest teilweise entlang eines fussseitigen Bereichs 76 der Einlage 7. Die zweite Kammer 72 ist im fussseitigen Bereich 76 der Einlage 7 angeordnet und wird sowohl proximal wie auch lateral von der ersten Kammer 71 umrahmt. Anders gesagt ist die zweite Kammer 72 in einer Ausnehmung der ersten Kammer 71 angeordnet, wobei hier ein Abstand zwischen den beiden Kammern 71, 72 ausgebildet ist. Die erste Kammer 71 entspricht einem Hohlraum, in welchen ein Fluid über die erste Ventilrichtung 73 eingeführt werden kann. Dadurch wird eine Versteifung der Einlage 7 im Bereich der ersten Kammer 71 erreicht. Beim Fluid handelt es sich vorzugsweise um Luft, welche in die erste Kammer 71 eingepumpt wird, währenddem die Einlage 7 in einer Stützschalenanordnung 1 angeordnet ist und ein Patient die Stützschalenanordnung 1 trägt. Auch bei der zweiten Kammer 72 handelt es sich um einen Hohlraum, welcher allerdings eine Vielzahl von Formkörpern 77 wie beispielsweise Styroporkugeln beinhaltet. Anders als bei der ersten Kammer 71 wird jedoch für eine Versteifung kein Fluid in die zweite Kammer 72 eingefüllt. Stattdessen wird die Versteifung der Einlage 7 im Bereich der zweiten Kammer 72 dadurch erreicht, dass ein Fluid über die zweite Ventileinrichtung 74 aus der zweiten Kammer 72 abgeführt wird. Beispielsweise kann eine Vakuumpumpe an die zweite Ventilanordnung 74 angeschlossen werden, welche die Luft aus der zweiten Kammer 72 absaugt. Auch hier trägt der Patient dabei die Stützschalenanordnung 1 mit Einlage 7, so dass die zweite Kammer 72 nach der Evakuierung die abgebildete Form des Körperteils aufweist. Wie aus der schematischen Abbildung in Figur 16 hervorgeht, kann eine erneute Verformbarkeit der zweiten Kammer 72 erreicht werden, indem ein Fluid wie z.B. Luft in die zweite Kammer 72 eingefüllt wird. Dadurch können sich die Formkörper 77 der zweiten Kammer 72 bewegen und verschieben und sich so erneut an das zu stützende Körperteil anpassen. Wird dann wiederum die Luft aus der zweiten Kammer 72 abgesaugt, so verbleiben die Formkörper 77 in ihrer neu eingestellten Position. Die zweite Kammer 72 kann also reversibel versteift werden. Gleiches gilt natürlich auch für die erste Kammer 71, aus welcher die eingelassene Luft abgelassen und alsdann erneut eingefüllt werden kann. Somit ermöglicht die Einlage 7 eine flexible, passgenaue und sichere Einbettung des Fusses, des Knöchels oder der Wade, wodurch Druckstellen vermieden und die Reduktion von Schmerzen und Schwellungen gefördert werden.

Damit Luft in die Kammern 71, 72 eingeführt beziehungsweise aus den Kammern 71, 72 abgeführt werden kann, müssen die Kammer aus einem gasdichten Material bestehen.

Eine flexible Anpassung der Einlage 7 kann weiter erreicht werden, indem eine oder mehrere Pumpen 78 in die Einlage 7 integriert werden. Beispielsweise kann die Einlage 7 eine Halterung 79 für eine Luftpumpe 78 zum Aufpumpen der ersten Kammer 71 aufweisen, welche die Form einer Seitentasche 79 aufweist und z.B. in die Einlage 7 eingenäht wird. Die Luftpumpe 78 kann eine manuell betätigbare Handpumpe sein, welche dann in diese Seitentasche 79 eingesteckt wird.

Vorteilhaft ist eine einzige Pumpe mit einem einzigen Blasebalg vorgesehen, welche von einem Pump- zu einem Saugmodus und umgekehrt umschaltbar ist. Zusammen mit der ersten und der zweiten Ventileinrichtung 73, 74 bildet diese Pumpe dann eine Pumpvorrichtung, welche je nach Modus der Pumpe zum Aufpumpen der ersten Kammer 71 bzw. zum Absaugen des Fluids aus der zweiten Kammer 72 dient. Die erste Ventileinrichtung 73 und die zweite Ventileinrichtung 74 sind dann entsprechend in unmittelbarer Nähe voneinander angeordnet bzw. über entsprechende Leitungen an die gemeinsame Pumpe gekoppelt.

Der Innenschuh weist vorteilhaft eine aus einem Polyesterstoff hergestellte Aussenschicht und eine aus einem Frotteestoff hergestellte Innenschicht auf. Zwischen der Aussen- und der Innenschicht ist bevorzugt ein Schaumstoff angeordnet. Die Kammern 71 bzw. 72 sind vorteilhaft zwischen dem Schaumstoff und der Innenschicht angeordnet.

Zusätzlich oder alternativ zum Innenschuh kann zudem ein in Bezug auf die Stützschalenanordnung sowie den allenfalls vorhandenen Innenschuh innenseitig angeordnetes Abstandsgewirke vorgesehen sein, welches zur Linderung von Druckstellen, zur verbesserten Luftzirkulation und/oder zur Aufnahme von abgesonderten Sekreten dienen kann.

**BEZUGSZEICHENLISTE**

| | | | |
|---|---|---|---|
| 1 | Stützschalenanordnung | 5 | Feststelleinrichtung |
| | | 51 | Feststellelement |
| 2 | Wadenteil | 52 | Führungsschiene |
| 21 | Gurtlaschen | 53 | Federelement |
| 22 | Befestigungsgurt | 54, 54' | Schenkel |
| 23 | Schlitz | 55 | Steg |
| 24 | Öffnungen | 56, 56' | Einrastelemente |
| 25 | Führungsnut | 57 | Rastkerben |
| 26 | Proximaler Bereich | 58, 58' | Einschnitt |
| 27 | Ausnehmung | 59 | Krümmung |
| 28, 28' | Öffnung | 510 | Anlagefläche |
| 29 | gebogener Bereich | 511 | Durchgangsöffnung |
| 290 | Ausbuchtung | 512 | Fixierelement |
| | | 513 | Durchgangsöffnung |
| 3 | Fussteil | 514 | Unterlagscheibe |
| 31 | Gurtlaschen | | |
| 32 | Öffnungen | 6, 6' | instelleinrichtung |
| 33 | Proximaler Bereich | 61, 61' | Drehknopf |
| 34, 34' | Öffnung | 62, 62' | Gelenkstange |
| 35 | Vorsprünge | 63,63' | Zapfen |
| 36 | Verbindungsstruktur | | |
| 37 | Proximales Ende | 7 | Innenschuh |
| 38 | Fersenriemen | 71 | Erste Kammer |
| 39 | Anschlag | 72 | Zweite Kammer |
| 310 | Zwischenraum | 73 | Ersten Ventileinrichtung |
| 311 | gebogener Bereich | 74 | Zweiten Ventileinrichtung |
| | | 75 | Proximaler Bereich |
| 4 | Sohlenteil | 76 | Fussseitiger Bereich |
| 41 | Ausnehmungen | 77 | Formkörper |
| 42 | Proximaler Bereich | 78 | Pumpe |
| 43 | Verlängerte | 79 | Seitentasche |
| | Verbindungsstruktur | | |
| 44 | Distaler Bereich | 8 | Drucksensoreinrichtung |
| | | 81, 81', 81", 81'" | Drucksensoren |
| 82, 82', 82", 82'" | Leitungen | FK | Federkraft |
| | | GK | Gegenkraft |
| 91 | Schienbeinteil | α | Beugewinkel |
| 92 | Fussrückenteil | LW | Hauptlängsachse von Wadenteil |
| | | FW | Hauptlängsachse von Fussteil |

## Patentansprüche

1. Stützschalenanordnung (1) umfassend:
ein Wadenteil (2);
ein Fussteil (3), welches gelenkig mit dem Wadenteil (2) verbindbar ist; und
eine Feststelleinrichtung (5) mit einer am Wadenteil (2) oder am Fussteil (3) angebrachten Führungsschiene (52) sowie mit einem Feststellelement (51),
wobei das Feststellelement (51), zum Einstellen eines Beugewinkels (α) zwischen dem Wadenteil (2) und dem Fussteil (3), entlang der Führungsschiene (52) verschiebbar und in einer gewünschten Position an der Führungsschiene (52) fixierbar ist,
**dadurch gekennzeichnet, dass**
die Feststelleinrichtung (5) zudem ein Federelement (53) umfasst, welches das Feststellelement (51) derart mit einer Federkraft (FK) beaufschlagt, dass ein Verschieben des Feststellelements (51) entlang der Führungsschiene (52) erleichtert oder erschwert ist.

2. Stützschalenanordnung (1) nach Anspruch 1, wobei die Führungsschiene (52) über eine Einraststruktur (57) und das Feststellelement (51) über eines oder mehrere Einrastelemente (56, 56') verfügt, und wobei das Feststellelement (51) bewegbar ist zwischen einer Verschiebeposition, in welcher das oder die Einrastelemente (56, 56') ausser Eingriff mit der Einraststruktur (57) stehen, und einer Fixierposition, in welcher das oder die Einrastelemente (56, 56') in Eingriff mit der Einraststruktur (57) stehen.

3. Stützschalenanordnung (1) nach Anspruch 2, wobei das Feststellelement (51) vom Federelement (53) mit einer Federkraft (FK) in Richtung der Verschiebeposition beaufschlagt wird.

4. Stützschalenanordnung (1) nach Anspruch 2, wobei das Feststellelement (51) vom Federelement (53) mit einer Federkraft (FK) in Richtung der Fixierposition beaufschlagt wird.

5. Stützschalenanordnung (1) nach einem der vorhergehenden Ansprüche, wobei die Feststelleinrichtung (5) weiter über zumindest ein Fixierelement (512), insbesondere in Form einer Fixierschraube, zur Fixierung des Feststellelements (51) in der gewünschten Position an der Führungsschiene (52) verfügt.

6. Stützschalenanordnung (1) nach einem der vorhergehenden Ansprüche, wobei das Fussteil (3) fersenseitig gebogen ausgebildet ist und mit diesem gebogenen Bereich (311) zumindest teilweise an einem entsprechend gebogenen Bereich (29) des Wadenteils (2) entlang bewegbar ist.

7. Stützschalenanordnung (1) nach einem der vorhergehenden Ansprüche, wobei die Führungsschiene (52) der Feststelleinrichtung (5) fersenseitig am Fussteil (3) angeordnet, insbesondere einstückig mit dem Fussteil (3) ausgebildet ist, oder
wobei die Führungsschiene (52) der Feststelleinrichtung (5) fersenseitig am Wadenteil (2) angeordnet, insbesondere einstückig mit dem Wadenteil (2) ausgebildet ist.

8. Stützschalenanordnung (1) nach einem der vorhergehenden Ansprüche, wobei das Feststellelement (51) zwischen dem Wadenteil (2) und dem Fussteil (3) angeordnet ist, und wobei das Feststellelement (51) vom Federelement (53) mit einer Federkraft (FK) in Richtung des Fussteils (3) oder des Wadenteils (2) beaufschlagt wird.

9. Stützschalenanordnung (1) nach einem der vorhergehenden Ansprüche, wobei die Führungsschiene (52) aussenseitig am Fussteil (3) angeordnet ist und das Wadenteil (2) innenseitig eine Führungsnut (25) zur geführten Aufnahme der Führungsschiene (52) aufweist.

10. Stützschalenanordnung (1) nach einem der vorhergehenden Ansprüche, wobei das Wadenteil (2) über mindestens einen Schlitz (23, 23') verfügt, welcher sich zumindest teilweise in das Wadenteil (2) hinein erstreckt, so dass eine flexible Anpassung des Wadenteils (2) an die Wade eines Patienten ermöglicht ist.

11. Stützschalenanordnung (1) nach einem der vorhergehenden Ansprüche, weiter umfassend ein Sohlenteil (4), welches mit dem Fussteil (3) verbindbar ist, wobei das Sohlenteil (4) über eine Drucksensoreinrichtung (8) zur Erfassung der Auftrittsbelastung des Sohlenteils (4) durch einen Patienten verfügt, und
wobei die Drucksensoreinrichtung (8) mindestens einen Drucksensor (81, 81', 81", 81''') aufweist, der zur Ausgabe eines Sensorsignals ausgebildet ist.

12. Stützschalenanordnung (1) nach Anspruch 11, weiter umfassend:
eine Auswerteeinrichtung, in welcher ein vorgegebener Maximalwert und/oder Maximalwertebereich der zulässigen Auftrittsbelastung speicherbar ist, und welche dazu ausgebildet ist, einen Sensorwert aus dem Sensorsignal des Drucksensors (81, 81', 81", 81''') zu ermitteln und den ermittelten Sensorwert mit dem vorgegebenen Maximalwert und/oder dem Maximalwertebereich zu vergleichen, und
eine Informationsübermittlungseinheit, welche dazu ausgebildet ist, bei einer Überschreitung des Maximalwerts und/oder des Maximalwertebereichs durch den ermittelten Sensorwert ein optisches und/oder akustisches Signal auszusenden.

13. Stützschalenanordnung (1) nach Anspruch 12, weiter umfassend;
eine Energieversorgungseinrichtung zur Versorgung der Auswerteeinrichtung und/oder der Informationsübermittlungseinheit mit Energie sowie einen Arbeitsschalter, wobei der Arbeitsschalter dazu ausgebildet ist, die Energieversorgung im Falle einer Auftrittsbelastung einzuschalten und die Energieversorgung, falls während einer gewissen Zeit keine Auftrittsbelastung detektiert wird, auszuschalten.

14. Stützschalenanordnung (1) nach einem der vorhergehenden Ansprüche, ausserdem umfassend ein Schienbeinteil (91), welches gemeinsam mit dem Wadenteil 2 dazu ausgebildet ist, den Unterschenkel des Patienten zu umwinden.

15. Einlage (7), insbesondere Innenschuh, für eine Stützschalenanordnung, insbesondere für eine Stützschalenanordnung (1) nach einem der vorhergehenden Ansprüche, umfassend:
eine erste Kammer (71);
eine zweite Kammer (72), welche eine Vielzahl von Formkörpern (77) beinhaltet,
eine erste Ventileinrichtung (73), mittels welcher ein Fluid in die erste Kammer einfüllbar ist, um die Einlage (7) in einem ersten Bereich zu versteifen; und
eine zweite Ventileinrichtung (74), mittels welcher ein Fluid aus der zweiten Kammer (72) abführbar ist, um die Einlage (7) in einem zweiten Bereich zu versteifen.

16. Einlage (7) nach Anspruch 15, wobei die erste Ventileinrichtung (73) und die zweite Ventileinrichtung (74) durch eine gemeinsame Pumpvorrichtung gebildet werden, welche von einem Pump- zu einem Saugmodus und umgekehrt umschaltbar ist.
